# EUROPEAN PATENT SPECIFICATION

(11) **EP 4 548 955 B1**
(45) Date of publication and mention of the grant of the patent: **18.11.2026**
(21) Application number: 25157196.4
(22) Date of filing: 23.06.2020
(51) Int. Cl.: A61M 39/10, A61J 1/00

(54) **AN ADAPTOR FOR A MEDICAL CONTAINER, A MEDICAL CONTAINER COMPRISING SAID ADAPTOR, AND A METHOD FOR MANUFACTURING THIS MEDICAL CONTAINER**
ADAPTER FÜR EINEN MEDIZINISCHEN BEHÄLTER, MEDIZINISCHER BEHÄLTER MIT DEM ADAPTER UND VERFAHREN ZUR HERSTELLUNG DIESES MEDIZINISCHEN BEHÄLTERS
ADAPTATEUR POUR RÉCIPIENT MÉDICAL, RÉCIPIENT MÉDICAL COMPRENANT LEDIT ADAPTATEUR ET PROCÉDÉ DE FABRICATION DE CE RÉCIPIENT MÉDICAL

(30) Priority: 28.06.2019 EP 19305878
(43) Date of publication of application: 07.05.2025
(62) Document number(s) of the earlier application(s) in accordance with Art. 76 EPC: 20733819.5 / 3 990 093
(73) Proprietor: Becton Dickinson France, 38800 Le Pont-de-Claix (FR)
(72) Inventor: RIVIER, Cédric, 38340 Voreppe (FR); DELEUIL, Nicolas, 38100 Grenoble (FR); JOUFFRAY, Sébastien, 38410 Saint Martin D'Uriage (FR)
(74) Representative: Germain Maureau

(56) References cited:
- EP-A1- 3 307 361
- DE-A1- 102008 061 415
- US-A1- 2014 276 213

## Description

The present invention relates to an adaptor for mounting onto a medical container, a medical container including this adaptor, and a method for manufacturing this medical container.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to a medical container of the invention, and the "proximal direction" is to be understood as meaning the opposite direction to said direction of injection, that is to say the direction towards the user's hand holding a container as for an injection operation.

Medical devices, for example injection devices such as pre-fillable or prefilled syringes, usually comprise a hollow body or barrel forming a container for a medical product. This body comprises a proximal end, provided with a flange allowing a user to place his or her fingers so as to exert a proximal pressure onto this flange, and a distal end, usually provided with a needle, said distal end being in the form of a longitudinal tip defining an axial passageway through which the medical product is expelled from the container. However, this longitudinal tip does not allow parenteral administration by itself and must either comprise a staked needle or an adaptor allowing the connection of the syringe to a connector such as a needle hub or an intravenous (IV) line. Basically, adaptors may be secured around the longitudinal tip of the syringe by gluing, screwing, snap-fitting or friction force. The connector is then mounted on the adaptor, for example by screwing.

There is an increasing need for individual traceability of the medical containers, such as injection devices, from the manufacturing process until at least the final use of said medical containers, typically the medical product injection.

It is known for example from document WO2017157784 a receptacle having a cylindrical lateral surface surrounded by a sequence of printed machine-readable unique identifier codes. These printed unique identifier codes allow tracking and tracing of each receptacle along a supply chain. However, these unique identifier codes are printed on an external side of the receptacle so that they may be removed or damaged for example during handling or use of the receptacle. Moreover, the unique identifier codes cover a portion of the receptacle so that they may have an impact on a customer visual inspection process.

It is further known from document US8872870 a method for glass-marking, wherein an array of readable marks may be formed by a laser on the external surface of a glass tube, for example for tracking purposes. However, the downsides of any laser marking methods are a possible damage to the glass material and an expensive manufacturing process. Besides, the laser-written array of mark requires having a visual access to the glass tube so as to be read, so that the reading operation cannot occur at any time. The laser-written array of mark may further have an impact on a customer visual inspection process.

Document US2014276213 further discloses an injection site information cap. Document DE102008061415 discloses a medical device hose connector, for connecting a respiration hose to a respiration device, that has electronic data storage element embedded between plastic parts that are cast together.

In this context, an object of the present invention is to provide an adaptor that alleviates the above-mentioned drawbacks by allowing easy individual identification of a medical container from the first step of the manufacturing step to the final use, typically the injection, or disposal step, with no impact on visual inspection and with few or no risks of being removed or damaged.

An aspect of the invention is an adaptor for a medical container having a distal tip, said adaptor comprising:
- a proximal part configured to be secured onto the distal tip of the medical container,
- a distal part configured to receive a connector, and
- a remotely readable electronic component configured to allow remote identification of the medical container when said adaptor is secured to the medical container, said remotely readable electronic component being at least partially embedded into the adaptor.

By electronic component embedded in the adaptor it should be understood that the material of the adaptor fully or at least partially encases the electronic component, so that the electronic component is protected from the external environment and therefore cannot be damaged or removed.

By remotely readable electronic component configured to allow remote identification of the medical container it is meant that the electronic component comprises electronically stored information that may be remotely read by a reading machine, such as a RFID reader, enabling identification of the medical container to which the adaptor of the invention is intended to be assembled.

The adaptor of the invention thus allows having an individual traceability of each medical container from the first steps of the manufacturing step to the final use of the medical container or to the disposal of said medical container. Besides, because the electronic component is at least partially encased in the adaptor, it is protected from removal or external damage that may occur due to the packaging, sterilization, storing, distribution or the use of the medical container. Furthermore, the electronic component being located inside the adaptor, there is no impact on customer visual inspection process. It is also contemplated that the electronic component permits remote and therefore easy identification of the medical container. The electronic component does not require a direct visual perspective from a reading machine so that the reading may occur at any time without a need to unpackage the medical container. Moreover, the electronic component is integrated inside the adaptor so that there is advantageously no additional thickness to the usually used adaptor or to the medical container barrel, and thus none change is required regarding the packaging or storing of the medical container.

In a preferred embodiment, the remotely readable electronic component is selected from the group consisting of a RFID tag, a ultra wide-band real-time location system (RTLS), a wifi RTLS and an infrared RTLS. Advantageously, the remotely readable electronic component is a RFID tag including a RFID chip and a RFID antenna, said RFID antenna preferably extending around a through-opening of the adaptor.

Preferably, the remotely readable electronic component is flush with an inner surface of the adaptor, more preferably with an inner surface of the proximal part of the adaptor.

This limits the risks that the remotely electronic component be damaged by the outside environment before adding the glue material. This also enables to mold the adaptor by one-shot molding.

Preferably, the proximal part has an inner surface that defines at least one annular space configured to accommodate a glue material, and said remotely readable electronic component is arranged so as to be covered by said glue material.

As a result, the remotely readable electronic component is embedded between the adaptor and the glue material. This enables to reduce manufacturing costs while efficiently protecting the remotely readable electronic component.

Accordingly, the adaptor is configured to be secured to the medical container by gluing.

Preferably, the remotely readable electronic component is completely located within the proximal part of the adaptor.

Preferably, the inner surface of the proximal part is provided with protrusions, and the RFID chip is located between two of said protrusions.

This limits the risk that the RFID chip be broken due to the pressure during the injection process.

The protrusions may divide the at least one annular space into several cavities.

Preferably, the proximal part comprises an annular ring, and the RFID antenna is located in this annular ring.

The annular ring may be provided at a distal end of the at least one annular space.

In an embodiment, the remotely readable electronic component is fully embedded in the adaptor.

This results in no change in the adaptor dimensions with regard to standard adaptor dimensions so as to avoid investment costs. In this embodiment, the adaptor is molded via a two-shot injection molding process.

When the remotely readable electronic component is fully embedded in the adaptor, said adaptor is configured to be secured to the medical container by gluing, press-fitting screwing or snap-fitting.

Another aspect of the invention is a medical container comprising a distal tip and an adaptor as above-described, said adaptor being mounted onto the distal tip of said medical container.

Preferably, the adaptor is secured to said distal tip by means of a glue material and the remotely readable electronic component of the adaptor is embedded between the adaptor and said glue material.

Alternatively, the adaptor is secured to said distal tip by press-fit, snapping or screwing means and the remotely readable electronic component of the adaptor is fully embedded within the adaptor.

Another aspect of the invention is a method for manufacturing a medical container as above-described, said method comprising the steps of:
- providing an adaptor as above-described,
- providing a distal tip of the medical container,
- connecting the adaptor to the distal tip by means of the proximal part of the adaptor.

Preferably, the method comprises at least one step chosen among the following steps:
- distributing a glue material between the proximal part of the adaptor and the distal tip;
- screwing the adaptor onto the distal tip; or
- snap fitting the adaptor onto the distal tip; or
- press-fitting the adaptor onto the distal tip.

The invention and the advantages arising therefrom will clearly emerge from the detailed description that is given below with reference to the appended drawings as follows:
- Figure 1 is a perspective view of an adaptor of the invention,
- Figure 2 is a cross-section view of an adaptor of the invention mounted onto the distal tip of a medical container,
- Figure 3 is a perspective view of a remotely readable electronic component of an adaptor of the invention.

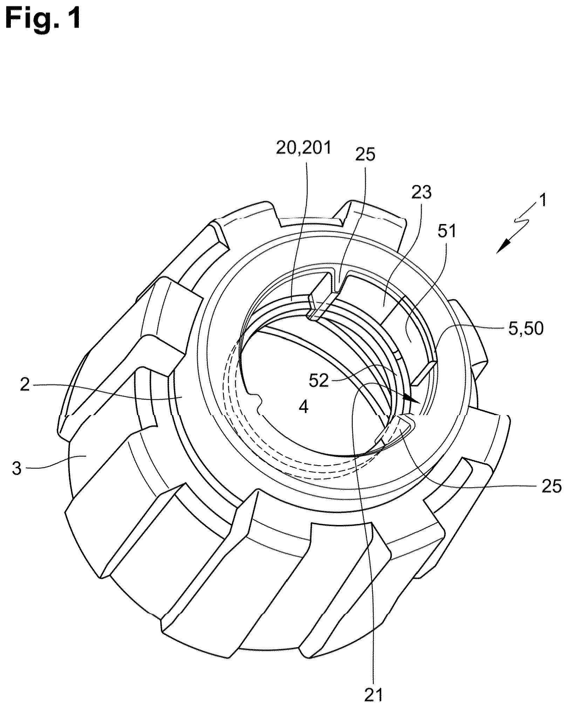

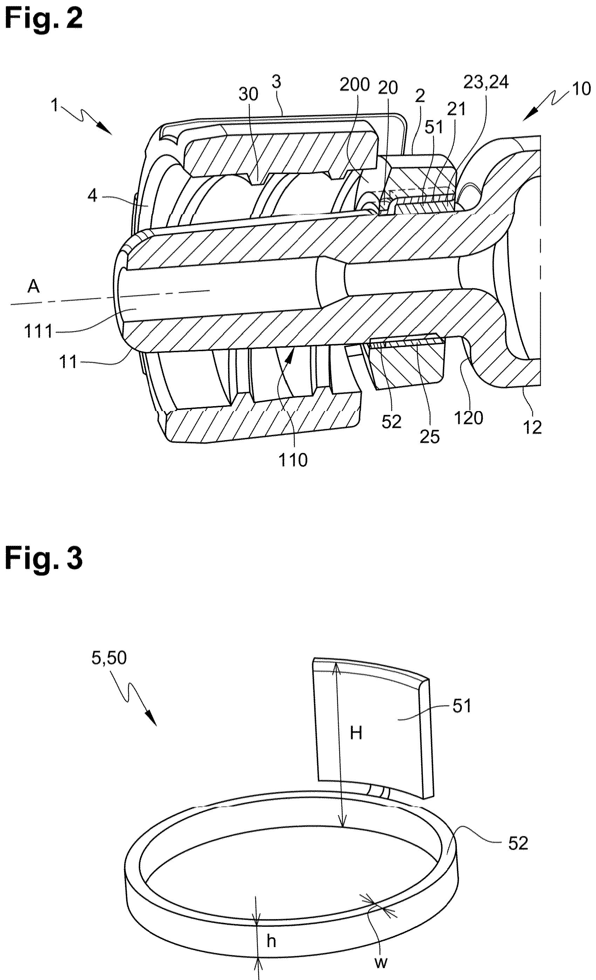

With reference to Figure 1 is shown an adaptor 1 according to an embodiment of the invention. The adaptor 1 is intended to be mounted onto a distal tip 11 of a medical container 10, as shown on Figure 2, more precisely onto an outer surface 110 of said distal tip 11. The outer surface 110 may be either cylindrical or distally tapered.

The adaptor 1 comprises a proximal part 2, which may be in the form of a mounting ring, configured to be secured to the medical container 10, and a distal part 3, which may be in the form of a connecting ring, configured to receive a connector (not shown), such as a needle hub or an intravenous (IV) line, in order to establish a reliable fluid communication between a passageway 111 of the distal tip 11 and said connector. The distal part 3 thus includes connecting means, such as an internal thread 30, in order to secure the connector to the distal tip 11. The connecting means may alternatively comprise a bayonet element, a snapping element or a press-fit element.

With reference to Figure 1, the adaptor 1 comprises a remotely readable electronic component 5 for remote identification of the medical container 10 by means of a remote reading machine. Advantageously, the remotely readable electronic component is a RFID tag 50, preferably a passive RFID tag 5. The RFID tag 50 may be read by a RFID reader, without requiring a direct pespective view on the adaptor 1.

With reference to Figure 3, the RFID tag 50 includes a RFID chip 51 and a RFID antenna 52. The RFID chip 51 may include at least a storage unit that stores a unique device identification (UDI), allowing the identification of the medical container 10.

The RFID tag 5 may have some or all of the following dimensions: an overall height H comprised between 0.5 and 10 mm, for example of about 2.4 mm; an antenna height h comprised between 0.1 and 8 mm, for example of about 0.5 mm ; an antenna width w comprised between 0.05 and 1 mm, for example of about 0.2 mm ; an antenna outside diameter comprised between 2 and 20 mm, for example of about 4.8 mm ; a RFID chip comprised between 0.5 x 0.5mm and 5 x 5 mm, for example of about 1.8 x 1.8 mm.

The remotely readable electronic component 5 may be overmolded in the adaptor 1.

The remotely readable electronic component 5 is at least partially embedded into the adaptor 1.

In a first embodiment, the remotely readable electronic component 5 is partially embedded in a material of the adaptor 1, for example during a one shot injection process. In this first embodiment, the remotely readable electronic component 5 is intended to be then covered by a glue material 24. As a result, the remotely readable electronic component 5 is thus partially embedded in the material of the adaptor 1, but however fully embedded between the adaptor 1, the glue material 24, and if needed the distal tip 11 of the medical container 10. According to this embodiment, the remotely readable electronic component 5 is preferably completely located in the proximal part 2 of the adaptor 1. Advantageoulsy, when the remotely readable electronic component 5 is a RFID tag 50 comprising a RFID chip 51 and an antenna 52, the chip 51 is fully embedded between the adaptor 1 and the glue material 24, while the antenna 52 may be left uncovered by the glue material 24, so that the antenna 52 contacts the distal tip 11 of the medical container 10 and is thus embedded between the adaptor 1 and the distal tip 11 of the medical container 10 when the adaptor 1 is mounted onto said distal tip 11. The antenna 52 may or may not be in contact with the glue material 24.

The glue material may be chosen between a hol-melt adhesive and glue, for example glue with an acrylate base.

In a second embodiment, the remotely readable electronic component 5 is fully embedded into the material of the adaptor 1. For instance, the remotely readable electronic component 5 may be overmolded in a two-shot injection process. More particularly, the remotely readable electronic component 5 is positioned as an insert into the mold. A first part of the adaptor 1 is formed by a first injection of a material and covers partially the remotely readable electronic component 5. A second part of the adaptor 2, that encapuslates totally the remotely readable electronic component 5 together with the first part, is then formed by a second injection step of a material that may be the same material as the first part or a different material. According to this embodiment, the remotely readable electronic component 5 may be located into the proximal part 2 and/or the distal part 3 of the adaptor 1.

Finally, in both of said first and second embodiments, when the adaptor 1 is mounted onto the distal tip 11 of the medical container 10, the remotely readable electronic component 5, especially the chip 51 of the RFID tag 50, is preferably fully embedded, either between the adaptor 1, the glue material 24 and/or the distal tip 11 of the medical container 10, or in the sole adaptor 1. The remotely readable electronic component 5 is thus protected from the outside environment. For instance, the adaptor 1 or medical container 10 may therefore be submitted to a sterilization process without damaging the remotely readable electronic component 5.

The adaptor 1 defines a central through-opening 4 extending through the proximal and distal parts 2, 3 along a longitudinal axis A. Said through-opening 4 defines a proximal portion and a distal portion, the proximal portion being configured to receive the distal tip 11, and the distal portion being configured to receive both the distal tip 11 and the connector connected around the distal tip 11. Therefore, the distal portion has a greater diameter than that of the proximal portion.

As shown on Figures 1 and 2, the RFID antenna 52 preferably extends around the through-opening 4 of the adaptor 1. As visible on Figure 1, the RFID antenna 52 extends all around the through-opening 4.

With reference to Figure 2, the proximal part 2 may advantageously comprise an annular ring 20 provided at a distal end of said proximal part 2. The annular ring 20 protrudes radially inwardly. Said annular ring 20 may define a distal shoulder 200 that separates the proximal and distal parts 2, 3. As shown on Figure 1, the annular ring 20 may further define a proximal shoulder 201 which is intended to serve as a stop for a glue material as will be described in further detail below.

As shown on Figure 1 or 2, the RFID antenna 52 is advantageously located in this annular ring 20.

The proximal part 2 comprises an inner surface 21 that may be provided with securing means in order to secure said proximal part 2 onto the outer surface 110 of the distal tip 11.

The securing means may comprise an annular space 23 configured to receive a glue material 24 in order to bond the adaptor 1 to the distal tip 11. The annular space 23 is delimited by the inner surface 21 of the proximal part 2, the annular ring 20, especially its proximal shoulder 201, and the outer surface 110 of the distal tip 11 when the adaptor 1 is mounted on the medical container 10. The annular space 23 may have an open proximal end to let in the glue material 24, while having a distal end which may be closed by the annular ring 20.

Preferably, the remotely readable electronic component 5, more precisely the RFID chip 51, partly delimits the annular space 23. The remotely readable electronic component 5 is substantially flush with the inner surface 21 of the proximal part 2, as illustrated on Figure 1. By a remotely readable electronic component 5 that is flush with the inner surface 21 it is meant that a part of said electronic component 5 is arranged at the same level as the inner surface 21 or may slightly protrude or form a recess with regard to said inner surface 21. The readable electronic component 5 is thus arranged so as to be covered the said glue material 24 received in the annular space 23.

The securing means may comprise one or several protrusions that may be in the form of longitudinal ribs 25 which may extend parallel to the longitudinal axis A. the longitudinal ribs 25 may be regularly distributed around on the circumference inner surface 21. The longitudinal ribs 25 may extend, preferably continuously, on the whole length of the inner surface 21. The longitudinal ribs 25 may contact the annular ring 20 or may be spaced from the annular ring 25. At least one of the longitudinal ribs 25 and the annular ring 20 may be in contact with the outer surface 110 of the distal tip 11 in order to increase the interference between the proximal part 2 of the adaptor 1 and said distal tip 11. The longitudinal ribs 25 may divide the annular space 23 into several cavities. For example, as illustrated on Figure 1, the proximal part 2 of the adaptor 1 comprises only three longitudinal ribs 25 in order to have an isostatic centering of the adaptor 1 onto the distal tip 11 of the medical container 10. The three longitudinal ribs 25 divide the annular space 23 in three cavities. It should be noted that at least one of these cavities, preferably each cavity, has an open proximal end so as to let in the glue material 24, while having a distal end which may be closed by the annular ring 20.

With reference to Figure 1, the RFID chip 51 is preferably located between two of said longitudinal ribs 25. The RFID chip 51 is thus preferably not embedded behind one of said protrusions. The RFID chip 51 may partly delimit one of the cavities formed by the longitudinal ribs 25.

The adaptor 1 may be made of a plastic material, more precisely of any rigid polymer adapted to medical use, such as high density polyethylene (PE), polypropylene (PP), polycarbonate (PC), acrylonitrile butadiene styrene (ABS), polyoxymethylene (POM), polystyrene (PS), polybutylene terephthalate (PBT), polyamide (PA), and combinations thereof. Preferably, the adaptor 1 is made of polcarbonate (PC). To simplify its manufacturing, the adaptor 1 is preferably made of a single piece of material. The adaptor 1 may however comprise two different material, for example when the adaptor 1 is made in a two-shot injection process. Preferably, the adaptor 1 is made of a light-transmitting material.

With reference to Figure 2, the invention also relates to the medical container 10 which may be a syringe. Said medical container 10 comprises a tubular barrel 12 that defines a reservoir for a medical product. The tubular barrel 12 is provided with the distal tip 11 that may protrude from a distal shoulder 120. The distal tip 11 may be cylindrical or distally tapered. The distal tip 11 includes an internal passageway 111 in communication with the reservoir. The tubular barrel 12 and the distal tip 111 are preferably made of glass. The medical container 10 may be provided with a piston and a plunger rod (not shown) so as to expel the medical product from the reservoir and through the passageway 111.

The medical container 10 also comprises an adaptor 1 having some or all of the above-described features. The adaptor 1 has its proximal part 2 mounted onto the distal tip 11.

In a preferred embodiment, the proximal part 2 of the adaptor 1 is secured to said distal tip 11 by means of the glue material 24. The remotely readable electronic component 5 is accordingly embedded between the adaptor 1 and said glue material 24. Due to this adhesive layer of glue material 24, the adaptor 1 may be bonded around the distal tip 11 without any need for a groove or a ring on the outer surface 110 of the distal tip 11. The distal tip 11 is thus devoid of such a groove or ring.

In another preferred embodiment, the proximal part 2 of the adaptor 1 may be secured to the distal tip 11 by press-fit, snapping or screwing means. The remotely readable electronic component 5 may be accordingly fully embedded within the adaptor 1.

The invention also relates to a method for manufacturing this medical container 10. The method comprises the steps of:
- providing an adaptor 1 having some or all of the above-described features, for example in a one-shot or two-shot injection process wherein the remotely readable electronic component 5 is partly or fully overmolded witin the adaptor 1,
- providing a distal tip 11 of the medical container 10,
- connecting the adaptor 1 to the distal tip 11 by means of the proximal part 2 of the adaptor 1.

As shown on Figure 2, the adaptor 1 may be connected onto the outer surface 110 of the distal tip 11.

Besides, the step of connecting the adaptor 1 to the distal tip 11 may comprise at least one step chosen among the following ones:
- distributing a glue material 24 between the proximal part 2 of the adaptor 2 and the distal tip 11, wherein said glue material 24 may be dispensed in the annular space 23; or
- screwing the adaptor 1 onto the distal tip 11; or
- snap fitting the adaptor 1 onto the distal tip 11; or
- press-fitting the adaptor 1 onto the distal tip 11.

When the method comprises the step of distributing a glue material 24 between the proximal part 2 of the adaptor 1 and the distal tip 11, it should be noted that the glue material 24 may be dispensed prior or posterior to the adaptor 1 being positioned around the distal tip 11. Moreover, the step of distributing the glue material 24 between the proximal part 2 of the adaptor 2 and the distal tip 11 may comprise the covering of the remotely readable electronic component 5 when said component is partially embedded in the adaptor 1, so that the remotely readable electronic component 5 is finally completely encased in the adaptor 1 plus the glue material 24.

## Claims

1. Medical container (10) comprising a distal tip (11) and an adaptor (1) mounted onto the distal tip (11), said adaptor (1) comprising:
- a proximal part (2) configured to be secured onto the distal tip (11) of the medical container (10),
- a distal part (3) configured to receive a connector, and
- a remotely readable electronic component (5) configured to allow remote identification of the medical container (10) when said adaptor (1) is secured to the medical container (10), said remotely readable electronic component (5) being at least partially embedded into the adaptor (1), **characterised in that** the remotely readable electronic component (5) is flush with an inner surface (21) of the adaptor (1), more preferably with an inner surface (21) of the proximal part (2) of the adaptor (1), wherein the adaptor (1) is secured to the distal tip (11) by means of a glue material (24) and the remotely readable electronic component (5) of the adaptor (1) is embedded between the adaptor (1) and said glue material (24).

2. The medical container (10) according to the preceding claim, wherein the remotely readable electronic component (5) is a RFID tag (50) including a RFID chip (51) and a RFID antenna (52), said RFID antenna (52) preferably extending around a through-opening (4) of the adaptor (1).

3. The medical container (10) according to any of the preceding claims, wherein the proximal part (2) has an inner surface (21) that defines at least one annular space (23) configured to accommodate a glue material (24), and said remotely readable electronic component (5) is arranged so as to be covered by said glue material (24).

4. The medical container (10) according to the preceding claim, wherein the adaptor (1) is configured to be secured to the medical container (10) by gluing.

5. The medical container (10) according to any of the preceding claims, wherein the remotely readable electronic component (5) is completely located within the proximal part (2) of the adaptor (1).

6. The medical container (10) according to claim 2 or any of claims 3-5 when dependent on claim 2, wherein an inner surface (21) of the proximal part (2) is provided with protrusions, and the RFID chip (51) is located between two of said protrusions.

7. The medical container (10) according to claim 2 or any of claims 3-6 when dependent on claim 2, wherein the proximal part (2) comprises an annular ring (20), and the RFID antenna (52) is located in this annular ring (20).

8. A method for manufacturing a medical container (10) as claimed in any of claims 1-7, said method comprising the steps of:
- providing the adaptor (1),
- providing a distal tip (11) of the medical container (10),
- connecting the adaptor (1) to the distal tip (11) by means of the proximal part (2) of the adaptor (1).

## Patentansprüche

1. Medizinischer Behälter (10), umfassend eine distale Spitze (11) und einen Adapter (1), der auf die distale Spitze (11) montiert ist, wobei der Adapter (1) umfasst:
- einen proximalen Teil (2), der konfiguriert ist, um an der distalen Spitze (11) des medizinischen Behälters (10) gesichert zu werden,
- einen distalen Teil (3), der konfiguriert ist, um einen Verbinder aufzunehmen, und
- eine fernlesbare elektronische Komponente (5), die konfiguriert ist, um eine Fernidentifizierung des medizinischen Behälters (10) zu ermöglichen, wenn der Adapter (1) am medizinischen Behälter (10) gesichert ist, wobei die fernlesbare elektronische Komponente (5) mindestens teilweise in den Adapter (1) eingebettet ist, **dadurch gekennzeichnet, dass**
die fernlesbare elektronische Komponente (5) mit einer Innenoberfläche (21) des Adapters (1), bevorzugter mit einer Innenoberfläche (21) des proximalen Teils (2) des Adapters (1) bündig ist,
wobei der Adapter (1) mittels eines Klebstoffmaterials (24) an der distalen Spitze (11) gesichert ist, und die fernlesbare elektronische Komponente (5) des Adapters (1) zwischen dem Adapter (1) und dem Klebstoffmaterial (24) eingebettet ist.

2. Medizinischer Behälter (10) nach dem vorstehenden Anspruch, wobei die fernlesbare elektronische Komponente (5) ein RFID-Tag (50) ist, das einen RFID-Chip (51) und eine RFID-Antenne (52) beinhaltet, wobei sich die RFID-Antenne (52) vorzugsweise um eine Durchgangsöffnung (4) des Adapters (1) herum erstreckt.

3. Medizinischer Behälter (10) nach einem der vorstehenden Ansprüche, wobei der proximale Teil (2) eine Innenoberfläche (21) aufweist, die mindestens einen Ringraum (23) definiert, der konfiguriert ist, um ein Klebstoffmaterial (24) aufzunehmen, und die fernlesbare elektronische Komponente (5) angeordnet ist, um von dem Klebstoffmaterial (24) abgedeckt zu werden.

4. Medizinischer Behälter (10) nach dem vorstehenden Anspruch, wobei der Adapter (1) konfiguriert ist, um durch Verkleben an dem medizinischen Behälter (10) gesichert zu werden.

5. Medizinischer Behälter (10) nach einem der vorstehenden Ansprüche, wobei sich die fernlesbare elektronische Komponente (5) vollständig innerhalb des proximalen Teils (2) des Adapters (1) befindet.

6. Medizinischer Behälter (10) nach Anspruch 2, oder einem der Ansprüche 3-5, wenn von Anspruch 2 abhängig, wobei eine Innenoberfläche (21) des proximalen Teils (2) mit Vorsprüngen bereitgestellt ist und sich der RFID-Chip (51) zwischen zwei der Vorsprünge befindet.

7. Medizinischer Behälter (10) nach Anspruch 2, oder einem der Ansprüche 3-6, wenn von Anspruch 2 abhängig, wobei der proximale Teil (2) einen ringförmigen Ring (20) umfasst und sich die RFID-Antenne (52) in diesem Ring (20) befindet.

8. Verfahren zur Herstellung eines medizinischen Behälters (10) nach einem der Ansprüche 1-7, wobei das Verfahren die Schritte umfasst zum:
- Bereitstellen des Adapters (1),
- Bereitstellen einer distalen Spitze (11) des medizinischen Behälters (10),
- Verbinden des Adapters (1) mit der distalen Spitze (11) mittels des proximalen Teils (2) des Adapters (1).

## Revendications

1. Récipient médical (10) comprenant une pointe distale (11) et un adaptateur (1) monté sur la pointe distale (11), ledit adaptateur (1) comprenant :
- une partie proximale (2) configurée pour être fixée sur la pointe distale (11) du récipient médical (10),
- une partie distale (3) configurée pour recevoir un raccord, et
- un composant électronique lisible à distance (5) configuré pour permettre l'identification à distance du récipient médical (10) lorsque ledit adaptateur (1) est fixé au récipient médical (10), ledit composant électronique lisible à distance (5) étant au moins partiellement incorporé dans l'adaptateur (1), **caractérisé en ce que** le composant électronique lisible à distance (5) affleure une surface interne (21) de l'adaptateur (1), plus préférablement une surface interne (21) de la partie proximale (2) de l'adaptateur (1),
dans lequel l'adaptateur (1) est fixé à la pointe distale (11) au moyen d'un matériau adhésif (24) et le composant électronique lisible à distance (5) de l'adaptateur (1) est incorporé entre l'adaptateur (1) et ledit matériau adhésif (24).

2. Récipient médical (10) selon la revendication précédente, dans lequel le composant électronique lisible à distance (5) est une étiquette RFID (50) comprenant une puce RFID (51) et une antenne RFID (52), ladite antenne RFID (52) s'étendant de préférence autour d'une ouverture traversante (4) de l'adaptateur (1).

3. Récipient médical (10) selon l'une des revendications précédentes, dans lequel la partie proximale (2) a une surface interne (21) qui définit au moins un espace annulaire (23) configuré pour recevoir un matériau adhésif (24), et ledit composant électronique lisible à distance (5) est agencé de manière à être recouvert par ledit matériau adhésif (24).

4. Récipient médical (10) selon la revendication précédente, dans lequel l'adaptateur (1) est configuré pour être fixé au récipient médical (10) par collage.

5. Récipient médical (10) selon l'une des revendications précédentes, dans lequel le composant électronique lisible à distance (5) est entièrement situé à l'intérieur de la partie proximale (2) de l'adaptateur (1).

6. Récipient médical (10) selon la revendication 2 ou l'une des revendications 3 à 5 lorsqu'elle dépend de la revendication 2, dans lequel une surface interne (21) de la partie proximale (2) est pourvue de saillies, et la puce RFID (51) est située entre deux desdites saillies.

7. Récipient médical (10) selon la revendication 2 ou l'une des revendications 3 à 6 lorsqu'elle dépend de la revendication 2, dans lequel la partie proximale (2) comprend une bague annulaire (20), et l'antenne RFID (52) est située dans cette bague annulaire (20).

8. Procédé de fabrication d'un récipient médical (10) tel que revendiqué dans l'une des revendications 1 à 7, ledit procédé comprenant les étapes de :
- fourniture de l'adaptateur (1),
- fourniture d'une pointe distale (11) du récipient médical (10),
- liaison de l'adaptateur (1) à la pointe distale (11) au moyen de la partie proximale (2) de l'adaptateur (1).
